Europäisches Patentamt

European Patent Office

Office européen des brevets

(19)

(11) Numéro de publication: **0 314 534**
**A1**

(12)

# DEMANDE DE BREVET EUROPEEN

(21) Numéro de dépôt: 88402230.2

(22) Date de dépôt: 05.09.88

(51) Int. Cl.⁴: **C 12 N 15/00**
C 12 N 7/00, C 12 N 5/00,
C 12 P 21/02, A 61 K 39/21,
A 61 K 39/395, G 01 N 33/569

(30) Priorité: 07.09.87 FR 8712396

(43) Date de publication de la demande:
03.05.89 Bulletin 89/18

(84) Etats contractants désignés:
AT BE CH DE ES FR GB GR IT LI LU NL SE

(71) Demandeur: TRANSGENE S.A.
16, rue Henri Régnault
F-92400 Courbevoie (FR)

INSTITUT PASTEUR
25, rue du Docteur Roux
F-75015 Paris (FR)

(72) Inventeur: Kieny, Marie-Paule
11, rue de Gascogne
F-67100 Strasbourg (FR)

Rautmann, Guy
16, rue Mariano
F-67000 Strasbourg (FR)

Guy, Bruno
15, rue de la Croix
F-67000 Strasbourg (FR)

Montagnier, Luc
21, rue de Malabry
F-92350 Le Plessis-Robinson (FR)

Alizon, Marc
71, rue du Cardinal Lemoine
F-75005 Paris (FR)

Girard, Marc
6, rue César Franck
F-75015 Paris (FR)

(74) Mandataire: Warcoin, Jacques et al
Cabinet Régimbeau 26, avenue Kléber
F-75116 Paris (FR)

Le titre de l'invention a été modifié (Directives relatives à l'examen pratiqué à l'OEB, A-III, 7.3)

(54) Vecteur d'expression des protéines du virus HIV-2, un des agents causal du SIDA.

(57) L'invention concerne un vecteur viral ou plasmidique d'expression d'une protéine du virus HIV-2 dans une cellule infectée ou transformée par ce vecteur, caractérisé en ce qu'il comporte au moins
- une partie du génome d'un virus hétérologue ou d'un plasmide
- une séquence de nucléotides codant pour l'une des protéines du virus HIV-2 (protéines gp ENV, protéines GAG, protéine F)
- ainsi que les élements assurant l'expression de ces protéines dans des cellules eucaryotes ou procaryotes.

EP 0 314 534 A1

**Description**

### VECTEUR D'EXPRESSION DES PROTEINES DU VIRUS HIV-2, UN DES AGENTS CAUSAL DU SIDA, CULTURE CELLULAIRE INFECTEE OU TRANSFORMEE PAR CE VECTEUR, PROTEINES OBTENUES, VACCIN ET ANTICORPS OBTENUS

La présente invention concerne un vaccin destiné à la prévention du SIDA.

Le syndrome d'immunodéficience acquise (SIDA) est une affection virale qui présente maintenant une importance majeure en Amérique du Nord, en Europe et en Afrique.

Les estimations récentes suggèrent que un ou deux millions d'Américains peuvent avoir été exposés au virus du SIDA. Les individus affectés présentent une immunodépression sévère et la maladie est, en général, fatale.

La transmission de la maladie s'effectue le plus souvent par contact sexuel, bien que les personnes utilisant des stupéfiants par voie intraveineuse représentent également un groupe à haut risque ; d'autre part, un grand nombre d'individus ont été infectés par ce virus après avoir reçu du sang ou des produits sanguins contaminés.

L'agent causal de cette affection est un rétrovirus. De nombreuses affections animales ont été attribuées aux rétrovirus, mais c'est seulement récemment que des rétrovirus affectant des hommes ont pu être décrits.

Alors que des rétrovirus des cellules T humaines de types I et II ont été impliqués comme agent causal de certaines leucémies des cellules T chez les adultes (HTLV : human T leukemia virus), le virus HIV (human immunodeficiency virus), est reconnu comme l'agent responsable du SIDA.

Le génome du rétrovirus HIV-1 a été caractérisé de façon très complète (Wain-Hobson et al., 1985 ; Ratner et al., 1985 ; Muesing et al., 1985 ; Sanchez-Pescador et al., 1985) et des informations sur la séquence génomique indiquent une relation étroite avec le groupe des lentivirus. Les lentivi rus, dont le prototype est le virus ovin Visna, sont les agents de maladies à progression très lente et qui présentent typiquement une période d'incubation prolongée. Le HIV et le virus Visna partagent de nombreuses similarités, en particulier dans leur tropisme pour le tissu neural.

Un virus apparenté à la famille des HIV, mais distinct du HIV-1, a été isolé récemment de malades du SIDA provenant surtout d'Afrique de l'ouest. Ce virus, appelé HIV-2, a été cloné et séquencé (Guyader et al., 1987) ; ces informations sur la séquence génomique confirment, malgré certaines divergences, que les virus HIV-1 et HIV-2 appartiennent à un même groupe et ont un même type de carte et d'organisation génomique.

Le nombre de malades contaminés par ce nouveau virus semble encore faible. Il est néanmoins indispensable dès à présent d'envisager une vaccination contre le SIDA comportant les protéines vaccinantes des virus HIV-1 et HIV-2, car l'importance épidémiologique du HIV-2 peut rejoindre celle du HIV-1.

HIV-1 et HIV-2 présentent tous deux un caractère pathogène à la différence de HTLV-4 décrit dans le brevet WO 87 02892. Mais HIV-2 présente des protéines dont la séquence diffère de celle des protéines de HIV-1 de sorte que les individus séropositifs pour HIV-2 présentent des anticorps qui ne peuvent pas réagir avec certaines protéines de HIV-1.

La séquence du HIV-2 fait apparaître une structure génétique très semblable à celle du HIV-1. En effet, outre les protéines GAG, POL et ENV, les 2 virus codent pour des protéines Q, F, R, TAT et ART uniques parmi les autres rétrovirus. Le HIV-2 code de plus pour une protéine putative X.

La séquence du produit du gène env révèle les caractéristiques attendues d'une glycoprotéine d'enveloppe transmembranaire. Le précurseur présente un poids moléculaire voi sin de 160 Kda et, comme c'est le cas pour HIV-1, est clivé en 2 protéines de 120 et 40 Kda.

Des anticorps dressés contre la protéine ENV gp160 et ses produits de clivage gp120, gp40 et gp32 dans certaines conditions (sur la gp32 voir Montagnier et al. 1987), sont communément détectés dans le sérum de patients infectés par HIV-2, et la glycoprotéine ENV représente l'antigène de suface majeur des virus du SIDA.

La protéine ENV apparaît ainsi comme un candidat prometteur pour développer une stratégie de vaccination parce qu'elle est exposée à la surface du virus. C'est pourquoi l'attention a été concentrée sur cette protéine et sur sa séquence codante.

Le gène gag code pour les protéines de structure internes du virion. Le messager issu de sa transcription est traduit sous la forme d'une protéine de 57 Kda (P55-57) et ce précurseur est alors maturé sous l'action d'une protéase spécifique codée par le gène pol. Le premier clivage endoprotéolytique génère une protéine de 40 Kda (P40) et de 12 Kda (P12) puis le deuxième clivage génère une protéine de 16 Kda (P16) et de 26 Kda (P26) ; l'ordre des cadres de lecture étant $H_2N$-P16-P26-P12-COOH. L'extrémité $NH_2$-terminale de la P55, confondue avec celle de la P16 peut, par analogie avec la P18 de HIV-1, être acylée par l'acide myristique. Ceci corrobore l'hypothèse selon laquelle la P16 peut servir de lien entre l'acide ribonucléique génomique et la membrane de la nucléocapside dans la particule virale. La P26 est le constituant majeur de la capside du virion. Le caractère fortement basique de la P12 suggère sa liaison intime avec les acides ribonucléiques viraux comme cela a été suggéré pour la P13 de HIV-1 (Wain-Hobson et al., 1985).

Il est aisé de détecter des anticorps dirigés contre les protéines codées par le gène gag dans les sérums des indi vidus infectés par HIV-2. Cette réponse immunitaire révèle le caractère fortement immunogène de ces différentes protéines On remarque également que lorsqu'un individu est séropositif pour HIV-2, une majorité des anticorps est dirigée contre la P26. Cette observation suggère, comme dans le cas du HIV-1, que les protéines codées par le gène gag sont une des cibles contre lesquelles il faut dresser une réponse immune, pour assurer une meilleure protection contre l'infection virale.

Plusieurs publications soulignent en effet l'importance des protéines de structure des virions dans l'induction des mécanismes d'immunité de type cellulaire. Parmi ces travaux il faut citer le cas du virus Influenza pour lequel une réaction CTL (lymphocytes T cytotoxiques) dirigée contre la nucléoprotéine (composante majeure de la capside du virion) peut se produire contre différents sous-types du virus alors qu'il n'existe pas de réaction immunitaire croisée entre sous-types pour les anticorps induits par les glycoprotéines de surface (hémagglutinine et neuraminidase). La structure primaire de ces glycoprotéines présente des zones variables dont les séquences peptidiques sont spécifiques à chaque sous-type. C'est cette divergence qui est responsable de l'absence de réaction immunitaire croisée. Une telle divergence existe aussi entre les différents isolats du HIV ; elle est maximale pour les séquences de la glycoprotéine ENV (jusqu'à 25 %) mais minimale (5 à 10 %) pour celles du gène gag (Starcick et al., 1986). C'est pourquoi l'utilisation des protéines codées par le gène gag pour stimuler une réponse immunitaire contre l'infection par le HIV est une des stratégies adoptées dans la présente invention.

La protéine F du HIV-1 est une protéine myristilée et dans certains isolats, elle est phosphorylée par la protéine kinase C. On a montré de plus que la protéine F régulait l'expression de l'antigène T4 et pouvait ainsi jouer un rôle important dans l'établissement et le maintien de l'infection causée par le HIV. L'induction d'une réponse immunitaire vis à vis de l'antigène F semble donc importante. La structure pri maire de la protéine F déduite de la séquence génomique du HIV-2 montre des similarités importantes avec la protéine équivalente du HIV-1. Il existe notamment un site potentiel de myristilation, essentiel pour l'ancrage dans la membrane, et un site potentiel de phosphorylation par la protéine kinase C en N-terminal. De plus des homologies importantes de séquence existent dans la partie C-terminale vraisemblablement la plus exposée au système immunitaire, la partie N-terminale étant ancrée dans la membrane par l'acide myristique hydrophobe. Il a donc semblé intéressant d'induire également une réponse immunitaire contre la protéine F du virus HIV-2.

La présente invention vise ainsi à fournir des moyens utiles pour la mise au point d'un vaccin contre le virus HIV-2. Tout au long de cette demande de brevet on entend désigner par virus HIV-2 aussi bien le virus tel qu'il est décrit dans la publication de Guyader et al. (1987) que d'éventuels mutants ponctuels ou des délétions partielles de ce virus, ainsi que les virus apparentés, en particulier les virus hybrides distincts de HIV-1 susceptibles d'induire un SIDA chez l'homme et les virus pouvant s'hybrider avec HIV-2. Le virus type de HIV-2 correspond aux plasmides mentionnés dans les exemples ci-après.

L'invention concerne, de façon générale, un vecteur viral ou plasmidique comportant une séquence de nucléotides codant pour l'une des protéines du virus HIV-2 et pouvant diriger l'expression de cette protéine dans une cellule eucaryote ou procaryote.

En d'autres termes l'invention a pour objet un vecteur viral ou plasmidique d'expression d'une protéine du virus HIV-2 dans une cellule infectée ou transformée par ce vecteur, et qui comporte au moins :
- une partie du génome d'un virus hétérologue ou d'un plasmide
- une séquence de nucléotides codant pour l'une des protéines du virus HIV-2
- ainsi que les éléments assurant l'expression de cette protéine dans des cellules eucaryotes ou procaryotes.

Parmi les vecteurs viraux utilisables, il s'agit bien entendu de virus hétérologues c'est-à-dire distincts de HIV-2 ; il faut citer plus particulièrement les poxvirus, virus tels que les adénovirus, les virus herpétiques et les baculovirus.

On préferera toutefois utiliser une partie du génome d'un poxvirus et plus particulièrement une partie du génome du virus de la vaccine (VV).

Le virus de la vaccine est un virus à ADN double brin qui a été utilisé très largement dans le monde entier pour contrôler et éradiquer la variole. Des développements techniques récents ont permis le développement de ce virus comme vecteur de clonage (Panicali et Paoletti, 1982) et des virus recombinants vivants ont permis d'exprimer des antigènes étrangers et même d'obtenir des immunisations contre différentes maladies virales ou parasitaires.

Ainsi, plusieurs groupes ont mis en évidence l'utilisation de recombinants de ce type pour exprimer l'antigène de l'influenza, de l'hépatite B et la glycoprotéine de la rage pour immuniser contre ces maladies (Smith et al., 1983 ; Panicali et al., 1983 ; Kieny et al., 1984). L'expression du gène env de HIV-1 dans le virus de la vaccine a également été décrite par Chakrabarti et al., 1986 ; Hu et al., 1986 et Kieny et al., 1986.

L'expression d'une séquence codant pour une protéine étrangère par le virus de la vaccine (VV) implique nécessairement trois étapes :

1) la séquence codante doit être alignée avec un promoteur de VV et être insérée dans un segment non essentiel de l'ADN de VV, cloné dans un plasmide bactérien approprié ;

2) les séquences d'ADN de VV situées de part et d'autre de la séquence codante doivent permettre des recombinaisons homologues entre le plasmide et le génome viral dans une cellu le réceptrice ; une double recombinaison réciproque conduit à un transfert de l'insert d'ADN du plasmide dans le génome viral dans lequel il est propagé et exprimé (Panicali et Paoletti, 1982 ; Mackett et al., 1982 ; Smith et al., 1983 ; Panicali et al., 1983) ;

3) l'expression de la séquence d'ADN intégrée dans le génome du VV recombinant dans une cellule appropriée.

Bien entendu, l'utilisation de ce type de vecteur implique souvent une altération partielle du génome du virus vecteur.

La présente invention concerne plus particulièrement un vecteur d'expression d'une protéine du virus HIV-2 dans une cellule infectée ou transformée par ce vecteur dans lequel la séquence de nucléotides codant pour l'une des protéines du virus HIV-2 est une séquence de nucléotides codant pour l'une des glycoprotéines (gp) de l'enveloppe du virus HIV-2.

Il convient de remarquer que les glycoprotéines (gp) de l'enveloppe du virus HIV-2 sont au nombre de 3, désignées par leur masse en Kda, à savoir la gp160, la gp120 et la gp40 (ou gp32) ; la première, gp160, est en fait le précurseur des deux dernières protéines.

Il est souhaitable d'exprimer ces trois protéines.

Les premiers essais conduits avec un vecteur viral dans lequel a été cloné le gène codant pour la protéine ENV totale ont conduit à proposer des modifications de ce gène pour améliorer l'immunogénicité des produits d'expression.

Comme dans le cas de HIV-1, on a constaté un relargage important de la protéine ENV dans les surnageants de culture (relargage qui se produit, probablement in vivo, dans les liquides circulants). Ceci peut être dû à un mauvais accrochage de la protéine dans la membrane cellulaire ; on sait en outre que la présentation des antigènes à la surface des cellules est très importante pour l'induction d'une réponse immunitaire avec le système vaccine. On propose donc de modifier le gène env de façon à améliorer l'ancrage de la glycoprotéine dans la membrane cellulaire.

Ceci est effectué en modifiant le gène env entre les séquences codantes de la gp120 et de la gp40 (ou gp32) pour supprimer les sites de clivage par les protéases situés entre la gp120 et la gp40 en particulier par la suppression du site KEKR (acides aminés 501-504).

La présente invention vise également l'expression des protéines GAG, et en particulier des protéines P57, P26 et P16.

Elle a donc aussi pour objet un vecteur viral ou plasmidique d'expression d'une protéine du virus HIV-2 dans une cellule infectée ou transformée par ce vecteur, dans lequel la séquence de nucléotides codant pour l'une des protéines du virus HIV-2 est une séquence de nucléotides codant pour au moins une des protéines GAG du virus HIV-2.

Enfin, la présente invention concerne l'expression du gène F. Elle concerne ainsi plus particulièrement un vecteur viral ou plasmidique d'expression d'une protéine du virus HIV-2 dans une cellule infectée ou transformée par ce vecteur, dans lequel la séquence de nucléotides codant pour l'une des protéines du virus HIV-2 est une séquence de nucléotides codant pour la protéine F du virus HIV-2.

Pour augmenter l'immunogénicité de la protéine F, il peut être intéressant d'ancrer cette dernière dans les membranes cellulaires. Suivant une caractéristique de l'invention, la séquence codant pour la protéine F est fusionnée en phase avec une séquence d'ancrage N-terminale, telle que celle de la protéine hémagglutinante (HA) du virus de la rougeole.

De façon générale quand on utilise, pour exprimer le gène en cause, le virus de la vaccine, il est préférable que le gène soit sous la dépendance d'un promoteur du virus de la vaccine, et on choisit de préférence le promoteur de la protéine 7,5 K. En outre, la séquence codante est clonée dans un gène non essentiel du virus de la vaccine. Dans la plupart des cas, il s'agira du gène TK qui pourra éventuellement servir de marqueur (TK⁻).

La présente invention concerne donc principalement l'utilisation de vecteurs viraux ou plasmidiques pour l'obtention des protéines codées par les gènes env, gag et F du virus HIV-2 ainsi que leurs produits de clivage dans des cultures cellulaires. Elle concerne également des cellules qui ont été infectées par un vecteur viral ou transformées par un plasmide selon l'invention ou bien qui peuvent contenir l'ADN recombinant correspondant ; parmi ces cellules, il faut citer plus particulièrement les cellules de mammifères, comme les cellules diploïdes humaines, les cellules Vero ou les cultures primaires. Bien entendu, il est possible de prévoir d'autres types de cellules, telles que les cellules d'insectes, les levures ou les bactéries.

Les protéines ainsi obtenues peuvent être utilisées après purification pour la réalisation de vaccins.

En particulier dans le cas de la glycoprotéine ENV, on peut exprimer une gp160 soluble, c'est-à-dire dépourvue de zone d'ancrage membranaire, et qui pourra être collectée dans les surnageants de cellules infectées afin de faire un "vaccin sous unités".

Il est également possible de prévoir l'utilisation directe des vecteurs viraux selon l'invention afin d'effectuer une vaccination, les glycoprotéines étant alors produites in situ et in vivo.

Il est intéressant de prévoir l'utilisation associée de plusieurs agents vaccinants administrés conjointement ou séparément, en particulier les agents vaccinants correspondant aux vecteurs exprimant la protéine ENV et une des protéines GAG ou la protéine F.

Il est également intéressant d'utiliser un vaccin mettant en oeuvre conjointement une protection contre HIV-1 et HIV-2.

Ces vaccins peuvent être obtenus soit par utilisation des virus recombinants correspondants décrits précédemment, vivants ou inactivés, ou bien en utilisant les produits de cultures de cellules infectées ou bien certains éléments de ces cultures.

Les vaccins en cause sont utilisables selon les techniques connues en particulier avec des adjuvants améliorant leur immunogénicité pour les produits des cultures cellulaires.

Les voies d'administration dépendent évidemment du type de vaccin choisi.

Enfin, la présente invention concerne également les anticorps dressés contre les protéines du HIV-2, obtenus par infection d'un organisme vivant avec un vecteur viral tel que décrit précédemment et récupération des anticorps induits après un temps déterminé.

Les antigènes obtenus comme les anticorps correspondants peuvent être utilisés dans des trousses de diagnostic permettant de dépister l'infection virale correspondante.

Les protéines recombinantes ainsi obtenues peuvent être utilisées dans des kits de diagnostic pour détecter les anticorps potentiels présents dans le sang des malades ayant été en contact avec le virus. Ces tests peuvent être mis en oeuvre selon des processus connus de l'homme de l'art, par exemple par ELISA, RIPA, "Westhern Blot" (immuno-empreinte).

Ces protéines peuvent également être utilisées pour la réalisation d'hybridomes et d'anticorps monoclonaux destinés à détecter la présence de virus dans des échantillons.

Les techniques mises en oeuvre pour l'obtention de ces protéines, les cultures cellulaires et les techniques de vaccination sont identiques à celles qui sont pratiquées actuellement avec les vaccins connus et ne seront pas décrites en détail.

<div align="center">METHODES</div>

Clonages
Maniatis et al., 1982.

Enzymes
Utilisées selon les prescriptions du fournisseur.

Mutagénèse localisée
Méthode dérivée de Zoller and Smith, 1983.

Transfert dans la vaccine
Kieny et al., 1984.
Seule différence : les cellules humaines 143B remplacent les cellules LMTK⁻.

Préparation du stock de virus
Les cellules primaires de poulet "germ free" sont infectées à 0,1 pfu/cellule pendant 4 jours à une température de 37°C (milieu MEM + 5 % NCS).

Purification du virus
On effectue une centrifugation du stock de virus ci-dessus pendant 15 minutes à 2500 tours (Rotor GSA Sorvall). Le surnageant est mis de côté. On reprend le culot dans un tampon RBS (Tris HCl 10 mM pH 7,4, KCl 10 mM, $MgCl_2$ 1 mM) pendant 15 minutes à 4°C. On effectue un broyage au potter, puis une centrifugation pendant 15 minutes à 2500 tours. Le surnageant est ajouté au précédent puis on effectue un deuxième broyage de la même façon.

Tous les surnageants sont déposés sur 10 ml de coussin de saccharose 36 % (p/v) (Tris 10 mM pH 8). On effectue une centrifugation pendant 2 heures à 14000 tours (Rotor SW28, Beckman).

Le culot est repris, dissocié et remis sur un deuxième coussin identique. Le 2ème culot est repris dans 5 ml PBS et chargé sur un gradient 20-40 % de saccharose (Tris 10 mM pH 8) (même rotor). On effectue une centrifugation pendant 45 minutes à 12000 tours.

On récupère la bande de virus - Elle est culottée par centrifugation pendant 1 heure à 20000 tours. Le culot est repris dans du Tris 10 mM pH 8.

Immunoprécipitations
On effectue une infection de cellules BHK21 (boîtes de 3 cm de diamètre, $10^6$ cellules par boîte, cultivées en G-MEM + 10 % FCS) à 0,2 pfu/cellule pendant 18 heures. Le milieu est décanté et remplacé par 1 ml de milieu sans méthionine et 10 μl de méthionine $^{35}$S (Amersham) par boîte.

On ajoute un excès de méthionine non radioactive après 2 heures.

A la fin du marquage, on effectue un grattage des cellules infectées, une centrifugation pendant 1 minute dans une centrifugeuse Eppendorf, une séparation des fractions surnageant et culot, un lavage du culot une fois en tampon PBS, puis une immunoprécipitation et un gel d'électrophorèse (selon Lathe et al., 1980).

Western blot
Cette technique de détection des anticorps dirigés contre les protéines du virus HIV-2 est dérivée de celle décrite dans le mode opératoire des Western blots LAV-BLOT® vendus par Diagnostics Pasteur.

Les exemples ci-après permettront de mettre en évidence d'autres caractéristiques et avantages de la présente invention.

Exemple 1 Construction du bactériophage M13 portant la séquence env.

Le plasmide pROD35, déposé à la Collection Nationale de Cultures de Microorganismes de l'Institut Pasteur, 28 Rue du Docteur Roux - 75015 PARIS, contient les 4,3 kb de l'extrémité droite du génome HIV-2 et comporte donc toute la séquence codante du gène env. (Ce plasmide porte le numéro de dépôt I-633).

Le fragment de restriction KpnI-KpnI (position 5304-9243) est inséré dans le site KpnI du bactériophage M13TG131, dans l'orientation telle que le sens de transcription soit dans le sens de transcription du gène de la β-galactosidase porté par M13 (M13TG1162).

Exemple 2 Construction du plasmide de transfert dans le virus de la vaccine portant le gène env.

Le site KpnI, situé en amont de la séquence codante de env, qui a été utilisé pour la construction du phage M13TG1162 est distant de 843 pb de l'ATG initiateur de env. Il faut donc créer un site de restriction à proximité de l'ATG. Ceci a été réalisé grâce à l'oligonucléotide :

```
5' CATCATACTCACAGATCTGGTGTAGG 3'
                 BglII
```

On introduit ainsi un site BglII dans le bactériophage M13TG1162, générant le phage M13TG1163. Le fragment de restriction BglII-BglII de M13TG1163 est ensuite inséré dans le site BamHI du plasmide pTG186POLY qui permet le transfert du gène env dans le virus de la vaccine (pTG2151).

Exemple 3 Suppression du signal stop présent dans la séquence codante de la gp40.

La séquence codante du gène env du plasmide pROD35 comporte un codon d'arrêt de traduction à la position 8304 (il s'agit là d'une particularité de plasmide pROD35 mais d'autres isolats comportent également un signal stop). En effet, un nucléotide T à cette position génère le codon TAG. La séquence correspondant à cette région a été effectuée sur d'autres clones et ceci établit que le nucléotide T doit être remplacé par un C. Il convient donc de muter le codon stop pour obtenir une séquence codant pour la totalité du gène env.

Ceci est réalisé grâce à une mutagénèse localisée à l'aide de l'oligonucléotide :

```
5' ATGGATCTGCTGGATAT 3'
           **
        CTATT
        GAT
        ←
        stop
```

\* positions non homologues

Le bactériophage obtenu est appelé M13TG1164.

Le fragment de restriction BglII-BglII de M13TG1164 est alors introduit dans le site BamHI du plasmide pTG186POLY pour générer le plasmide pTG2152.

Exemple 4 Construction du plasmide portant le gène env non clivé.

La protéine gp120 et la protéine gp40 sont générées par clivage protéolytique de la gp160. La gp120 est rapidement relarguée dans le milieu de culture. Il serait donc intéressant d'obtenir une protéine ENV non clivée. En effet, les protéines solubles sont peu immunogènes dans le système d'expression vaccine.

Pour ce faire, on modifie dans M13TG1164 la séquence nucléotidique du gène env dans la partie correspondant au site de clivage (position 7635-7648) grâce à l'oligonucléotide suivant :

5' GAGCAGAGGAGTAGTTGATATCTTGTGTAGGTGCGAA 3'.

La mutagénèse permet l'introduction d'un site de restriction EcoRV qui permet l'identification des clones mutants et la nouvelle séquence est la suivante :

```
                     T    K    E    K    R
clone original : CCT ACA  AAA  GAA  AAA  AGA  TAC
                          *         *   **   **
muté           : CCT ACA  CAA  GAT  ATC  AAC  TAC
                     T    Q    D    Q    N
                               EcoRV
```

Le bactériophage généré est appelé M13TG1165.

Le fragment de restriction BglII est ensuite cloné dans le site BamHI du plasmide de transfert pTG186POLY pour générer le plasmide pTG2158.

Exemple 5 Construction d'un bactériophage M13 contenant la séquence codante du gène gag.

Le plasmide pROD27-5', déposé à la Collection Nationale de Cultures Microorganismes de l'Institut Pasteur, 28 Rue du Docteur Roux - 75015 PARIS, contient un fragment de restriction EcoRI qui comporte l'extrémité gauche du génome du virus HIV-2. (Ce plasmide porte le numéro de dépôt I-626).

Le fragment de restriction BglI-EcoRI (position 502-2658) est inséré entre les sites BamHI et EcoRI du bactériophage M13TG131, après remplissage des extrémités BglI et BamHI à la polymérase de Klenow.

On génère de cette fa on le phage M13TG1155 qui contient toute la séquence du gène gag.

Exemple 6 Construction d'un plasmide permettant l'expression des protéines codées par le gène gag.

Le fragment de restriction BglII-EcoRI du phage M13, TG1155 est cloné entre les sites BamHI et EcoRI du plasmide de transfert pTG186POLY générant pTG2112. Le site BglII dans M13TG1155 provient du polylinker du bactériophage vecteur.

Exemple 7 Construction d'un plasmide permettant l'expression de la P26.

Le fragment de restriction PstI-EcoRI du bactériophage M13TG1155 est cloné entre les sites PstI et EcoRI du bactériophage M13TG130. Une mutagénèse localisée réalisée avec 2 oligonucléotides permet d'introduire simultanément un site de restriction BglII et un codon ATG à l'extrémité 5' (position 951) de la séquence codant pour la P26, et un site de restriction SstI et un codon STOP à l'extrémité 3' (position 1641). Les oligonucléotides sont les suivants :

```
                 BglII        M    G    P
    5' GGAGGAAATTACAGATCTATAATGGGTCCAGTGCAACAT 3'
                   -3
               * * * * * * * * * * * * * *

         M  Stop  SstI
    5' GCTAGATTAATGTGAGAGCTCCTGAAAGAGGTC 3'
               **        **
```

* positions non homologues à la séquence parentale.

On génère ainsi le bactériophage M13TG1158 à partir du phage M13TG1157.

Le fragment de restriction BglII-SstI de M13TG1158 est ensuite cloné entre les sites BamHI et SstI du plasmide de transfert pTG186POLY (pTG2111).

Exemple 8 Construction d'un plasmide permettant l'expression de la P16.

Dans le bactériophage M13TG1155, un site BglII est positionné en aval de l'ATG initiateur du gène gag (et donc de la séquence codant pour la P16).

Il faut donc réaliser une mutagénèse localisée afin d'introduire un codon STOP et un site de restriction à l'extrémité 3' de la séquence codant pour la P16. Ceci est réalisé grâce à l'oligonucléotide suivant :

(AGT)

5' CCGCCTACTGTTGAATTCAGTAATTTCCTCCC 3'
        EcoRI

On génère ainsi le bactériophage M13TG1156.

Le fragment de restriction BglII-EcoRI de M13TG1156 est ensuite inséré entre les sites de restriction BamHI et EcoRI du plasmide de transfert pTG186POLY, générant le plasmide pTG2110.

Exemple 9 Construction du plasmide pTG1198 contenant le gène F.

La séquence codant pour la protéine F du HIV-2 provient d'un cADN cloné dans le plasmide PSPE2 (Institut Pasteur, Paris). Le fragment de 950 pb obtenu par coupure au moyen de l'enzyme PvuII et contenant le gène F (nucléotides 8429 à 9378) est cloné dans le phage M13MP8 ouvert au site SmaI. Le phage obtenu est le M13TG1152. Afin de créer un site BglII en amont de l'ATG d'initiation de traduction du gène F, une mutagénèse a été conduite au moyen de l'oligonucléotide de séquence :

5' ACTCGCACCCATATTAGATCTAGGCTGTTCTAAGTC 3'
        BglII

Le phage résultant est le M13TG1153. Le fragment BglII-SalI de M13TG1153 contenant la séquence codante du gène F est cloné dans un plasmide de transfert, pTG186POLY, afin d'effectuer le transfert dans le virus de la vaccine comme précédemment décrit. Le plasmide résultant est le pTG1198.

Exemple 10 Construction du plasmide pTG2157 contenant le gène F précédé de la séquence codant pour la zone d'ancrage de la protéine HA du virus de la rougeole.

Le plasmide pTG1169 a été déposé à la Collection Nationale de Cultures de Microorganismes de l'Institut Pasteur le 3 avril 1987 sous le n° I-657.

Le fragment PstI-BamHI du plasmide pTG1169 (voir brevet français 87.09629) contenant la séquence codant pour l'hémagglutinine (HA) du virus de la rougeole est cloné dans le phage M13TG131 (même brevet) ouvert aux mêmes sites. Le phage résultant est le M13TG1159.

Un site BamHI est créé juste en aval de la séquence codant pour la zone d'ancrage hydrophobe de l'HA de la rougeole dans le M13TG1159 à l'aide de l'oligonucléotide :

5' CTCTGCGGTGTAGATGGATCCCCGATGAAGTCTAAT 3'
        BamHI

Le phage résultant est le M13TG1160.

Le fragment PstI-BamHI de M13TG1160 de 200 paires de bases contenant le fragment de séquence codant pour HA (zone hydrophobe) est cloné dans pTG186POLY ouvert aux mêmes sites. Le plasmide résultant est le pTG2155. Ce plasmide permet de cloner dans le site BamHI situé en aval de la zone d'ancrage de HA un gène étranger possédant en phase un site BamHI ou BglII (voir schéma) (dans ce cas, le codon GGA ou AGA est codant).

```
  Pst1                      BamHI
    ATG
  ┌─────────────────────────────────┐
──┤         ▨▨▨▨▨          ├───────────  pTG2155
  └─────────────────────────────────┘
            zone d'ancrage
  └──────────────┬──────────────┘
     Fragment N-terminal
     HA rougeole
```

Le fragment BglII de M13TGI153 (décrit plus haut) et contenant le gène F du HIV-2 est cloné dans l'orientation codante dans le pTG2155 ouvert par BamHI. Le plasmide résultant contenant le gène F fusionné avec la zone d'ancrage de HA est le pTG2157.

La séquence codante de la protéine F hybride est transférée dans le virus de la vaccine. Le virus recombinant ainsi créé est le VV.TG.F.HIV-2.2157.

Exemple 11 Introduction des gènes codant pour les protéines du HIV-2 dans le génome du virus de la vaccine et isolement de virus recombinés.

La stratégie décrite par Smith et al. (1983) repose sur la recombinaison génétique qui se réalise in vivo entre les séquences homologues des génomes du virus de la vaccine, dans la cellule infectée. Ce phénomène permet le transfert d'un fragment d'ADN cloné dans un plasmide vers le génome viral. L'utilisation du gène de la thymidine kinase (TK) du virus de la vaccine permet non seulement de provoquer l'intégration de l'ADN étranger dans le locus de ce gène mais aussi de disposer d'un marqueur phénotypique pour la sélection des virus recombinants qui sont devenus TK⁻.

Les virus TK⁻ peuvent être sélectionnés par étalement sur une lignée cellulaire TK⁻, en présence de 5-bromo-déoxy uridine (5BUDR) (Mackett et al., 1982). Un virus TK⁻ peut y répliquer son ADN normalement et former des plages visibles.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression de son génome. L'ADN de VV purifié est non infectieux.

Afin de générer les recombinants, il est nécessaire d'effectuer simultanément l'infection cellulaire avec du virion VV et une transfection avec le segment d'ADN étranger cloné qui porte une zone d'homologie avec l'ADN de la vaccine. Toutefois, la génération des recombinants est limitée à la petite proportion des cellules qui sont compétentes pour la transfection par l'ADN.

L'utilisation comme virus infectieux vivant d'un mutant thermosensible (ts) de la vaccine qui est incapable de se propager à une température non permissive de 39,5°C (Drillien et Spehner, 1983) diminue le bruit de fond constitué par les virus non-recombinants. Lorsque les cellules sont infectées par un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun virus ne se multipliera dans les autres cellules, en dépit du fait qu'elles ont été infectées. Si un plasmide recombinant contenant un fragment de l'ADN de vaccine est inclus dans le mélange de transfection, à la concentration appropriée, avec l'ADN du type sauvage, il est également possible d'obtenir qu'il participe à la recombinaison homologue avec l'ADN de la vaccine, dans les cellules compétentes.

Des monocouches de cellules primaires de fibroblastes d'embryons de poulets (CEF) sont infectées à 33°C avec VV-Copenhague ts7 (0,1 ufp/cellule) et transfectées avec un coprécipité au phosphate de calcium de l'ADN du virus de type sauvage VV-Copenhague (50 ng/$10^6$ cellules) et le plasmide recombinant (50 ng/$10^6$ cellules).

Après incubation pendant 2 heures à une température de 33°C, les cellules sont incubées pendant 48 heures à 39,5°C, température qui ne permet pas le développement du virus ts. Des dilutions de virus ts⁺ sont utilisées pour infecter des monocouches de cellules humaines 143B-TK⁻ à 37°C en présence de 5BUDR (150 μg/ml). Différentes plages de virus TK⁻ sont obtenues à partir de ces cellules qui ont reçu le plasmide recombinant, tandis que les cultures contrôles sans plasmide ne montrent pas de plages visibles. Les virus TK⁻ sont ensuite sous-clonés par une deuxième sélection en présence de 5BUDR.

Une double recombinaison réciproque entre les plasmides de transfert et le génome de VV aboutit à l'échange du gène TK portant l'insert avec le gène TK du virus, les recombinants devenant ainsi TK⁻.

Les ADN purifiés à partir des différents virus recombinants TK⁻ sont digérés par HindIII et soumis à une électrophorèse sur gel d'agarose. Les fragments d'ADN sont transférés sur un filtre de nitrocellulose selon la technique décrite par Southern (1975). Le filtre est ensuite incubé avec les plasmides utilisés pour le transfert, préalablement marqués avec l'isotope $^{32}$P. Le filtre est lavé et autoradiographié. Après le développement, on observe sur le film la présence de fragments dont la taille atteste du transfert des gènes concernés dans le génome de la vaccine.

Pour chacun des plasmides, un virus recombinant a été sélectionné et dénommé VV.TG.HIV-2 avec la même numérotation que les plasmides . Les virus recombinants suivants ont ainsi été générés :

VV.TG.HIV-2-2151 (ENV gp120-gp32)
VV.TG.HIV-2-2152 (ENV gp120-gp40)
VV.TG.HIV-2-2158 (ENV non clivé)
VV.TG.HIV-2-2112 (GAG)
VV.TG.HIV-2-2111 (P26)
VV.TG.HIV-2-2110 (P16)
VV.TG.HIV-2-1198 (F)
VV.TG.HIV-2-2157 (F membranaire)

Exemple 12 Immunoprécipitation des protéines synthétisées par le virus recombinant VV.TG.F.HIV-2-1198.

Pour mettre en évidence l'expression du gène F du HIV-2 à partir du virus de la vaccine recombinant, on infecte des cellules de rongeur, BHK21, qui sont cultivées dans un milieu G-MEM + 5 % de sérum de veau foetal avec ledit recombinant VV.TG.F.HIV-2-1198.

Une monocouche semi-confluente ($10^6$ cellules) est infectée avec 0,2 ufp/cellule et incubée pendant 18 heures.

Le milieu est ensuite éliminé et on ajoute, soit un milieu à faible teneur en méthionine (1 ml pour $10^6$ cellules) supplémenté avec 10 µl/ml de méthionine $^{35}$S (5 mCi/300 µl), soit 100 µCi/ml d'acide myristique marqué au tritium ($^3$H) (Amersham), en milieu MEM.

Les cellules sont incubées à 37°C et les protéines marquées sont collectées par centrifugation. Après séparation en culot et surnageant, les protéines sont incubées avec un sérum appartenant à des patients séropositifs vis à vis des antigènes du HIV-2.

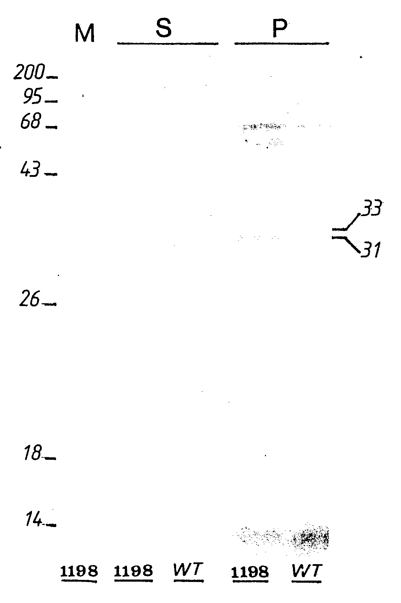

Les protéines réagissant avec le sérum sont récupérées par adsorption sur une résine protéine A-sépharose, étalées par électrophorèse sur un gel de polyacrylamide SDS et autoradiographiées selon une technique décrite par Lathe et al. (1980). Sur 4 sérums HIV-2(+) testés, un seul a permis d'immunoprécipiter spécifiquement la protéine F du HIV-2. Les immunopréciptés obtenus en utilisant ce sérum sont apparents sur l'autoradiographie de la figure 1 sur laquelle :

M = marquage à l'acide myristique $^3$M
S et P = marquage à la $^{35}$S méthionine
(S = surnageant
(P = culot cellulaire

Les poids moléculaires sont en kilodaltons.

Ces immunoprécipités font apparaître en marquage à la $^{35}$S méthionine 2 protéines spécifiques migrant à 31 et 33 kda, tant dans le culot cellulaire que dans le surnageant de culture. Le marquage à l'acide myristique tritié ne laisse apparaître qu'une seule protéine migrant à 31 Kda. La différence entre le poids théorique (28 Kda) et le poids observé de la protéine F du HIV-2 avait déjà été observée pour la protéine F du HIV-1. Comme dans ce dernier cas, la bande inférieure correspond à la forme myristilée.

Exemple 13

Production de la p16 du HIV2 par une souche E.coli

La délétion du fragment de restriction EcoRI contenant les cadres de lecture des protéines p26 et p12 du gène GAG du M13TG1156 a donné le plasmide M13TG1991.

L'oligonucléotide synthétique 5'-TCTGGCGCCCATAGATCTCAATCGGCTACC-3' permet l'introduction d'un site de restriction BglII immédiatement en amont de l'ATG initiateur de la p16 par mutagénèse dirigée du M13TG1991. Le fragment de restriction BglII-EcoRI du M13TG1992 obtenu après la mutagénèse est cloné dans le vecteur d'expression procaryote PTG959 - digéré par les enzymes BamHI et EcoRI. Le plasmide pTG3947 obtenu par ce clonage est utilisé pour transformer la souche E.coli TGE201 et l'on peut induire la production de la p16 par le maintien de la culture bactérienne à la température de 42°C.

Exemple 14

Production de la p26 du HIV2 par E.coli

L'oligonucléotide synthétique 5'-GGAGGAAATTACTCTAGATCTATGGCACCAGTGCAACAT-3' permet l'introduction d'un site de restriction BglII et des acides aminés méthionine et alanine à l'extrémité NH$_2$ terminale du cadre de lecture de la p26 de M13TGII58 pour donner le M13TG1994. Une nouvelle mutagénèse avec l'oligonucléotide synthétique 5'-TAATGTGAGAATTCCTGAAAGA-3' positionne un codon STOP et un site pour l'enzyme de restriction EcoRI à l'extrémité COOH terminale du cadre de lecture de la p26 pour donner le M13TG1995. Le fragment BglII-EvoRI contenant le cadre de lecture de la p26 est cloné dans le vecteur d'expression procaryote PTG959 traité par les enzymes BglII et EcoRI pour générer le plasmide pTG3948. La souche E.coli TGE901 est transformée par ce plasmide et la production de la p26 est induite dans les bactéries par le maintien de la culture à la température de 42°C.

Exemple 15

Production de la p57 du HIV2 par E.coli

L'oligonucléotide 5'-TCTCGCGCCCATAGATCTCAATCGGCTACC-3' permet l'introduction d'un site pour l'enzyme de restriction BglII immédiatement en amont de l'ATG initiateur du cadre de lecture de la p57 du M13TG1155 par mutagénèse dirigée. Le M13TG1993 issu de cette mutagénèse est traité par les enzymes BglII et EcoRI (digest partiel). Le fragment de restriction contenant le cadre de lecture de la P57 est cloné dans le vecteur d'expression procaryote pTG959 traité par les enzymes BglII et EcoRI pour donner le plasmide pTG3949. La souche E.coli TGE901 est transformée par ce plasmide et la production de la p57 est induite par une culture des bactéries à 42°C.

Exemple 16

Expression de la protéine Q dans le virus de la vaccine

Le fragment de restriction BamHI-HindIII du plasmide pROD35 (Guyader et al., 1987) a été sous cloné dans le bactériophage M13TG131 ouvert aux mêmes sites. Un site EcoRI a été généré en amont du site d'initiation de la traduction du gène Q. Pour ce faire a été utilisé l'oligonucléotide TG1679 deséquence 5'-CTCCATAGTCTCGAATTCTCTTGGCTTTCC-3'. Le fragmnet EcoRI obtenu et contenant le gène Q du HIV2 a été sous cloné dans le plasmide PTG186, et dans un deuxième temps transféré dans le virus de la vaccine selon une méthode précédemment décrite (Kieny et al., 1984). Le recombinant obtenu est le VV.TG.QHIV2-3142. Comme nous ne disposons pas actuellement d'anticorps spécifiques de la protéine Q du HIV2, l'intégration du gène Q dans le génome viral a été vérifiée par Southern Blot. Les résultats obtenus montrent une intégration correcte.

## REFERENCES

Chakrabarti, S., Robert-Guroff, M., Wong-Staal, F., Gallo, R.C. and Moss, B. Nature 320, 535-540 (1986).
Drillien, R., Spehner, D., Virology 131, 385-393 (1983).
Guyader, M., Emerman, M., Sonigo, P., Clavel, F., Montagnier, L. and Alizon, M, Nature 326, 662-669 (1987).
Hu, S.L., Kosowski, S.G. and Dabrymple, J.M. Nature 320, 537-540 (1986).
Kieny, M.P., Lathe, R., Drillien, R., Spehner, D., Skory, S., Schmitt, D., Wiktor, T., Koprowski, H. and Lecocq, J.P. Nature 312, 163-166 (1984).
Kieny, M.P., Rautmann, G., Schmitt, D., Dott, K., Wain-Hobson, S., Alizon, M., Girard, M., Chamaret, S., Laurent, A., Montagnier, L. and Lecocq, J.P. Biotechnology 4, 790-795 (1986).
Lathe, R., Hirth, P., Dewilde, M., Harford, N. and Lecocq, J.P. Nature 284, 473-474 (1980).
Mackett, M., Smith, G.L. and Moss, B. Proc. Natl. Acad. Sci. USA 79, 7415-7419 (1982).
Montagnier, L. and Alizon, M. Ann. Inst. Pasteur 138, 3-12 (1987).
Muesing, M.A., Smith, D.H., Cabradilla, C.D., Benton, C.V., Lasky, L.A. and Capon, D.J. Nature 313, 450-458 (1985).
Panicali, D. and Paoletti, E. Proc. Natl. Acad. Sci. USA 79, 4927-4931 (1982).
Panicali, D., Davis, S.W., Weinberg, R.L., Paoletti, E. Proc. Natl. Acad. Sci. USA 80, 5364-5368.
Ratner, L., Haseltine, W., Patarca, R., Livak, K.J., Starcich, B., Josephs, S.F., Doran, E.R., Rafalski, J.A., Whitehorn, E.A., Baumeister, K., Ivanoff, L., Petterway Jr., S.R., Pearson, M.L., Lautenberger, J.A., Papas, T.S., Ghrayeb, J., Chang, N.T., Gallo, R.C. and Wong-Staal, F. Nature 313, 277-284 (1985).
Sanchez-Pescador et al., Science 227, 484-492 (1985).
Smith, G.L., Mackett, M., Moss, V. Nature 302, 490-495 (1983).
Smith, G.L., Murphy, B.R., Moss, B. Proc. Natl. Acad. Sci. USA 80, 7155-7159 (1983).
Starcich, B., Hahn, B.H., Shaw, G.M., McNeely, P.D., Modrow, S., Wolf, H., Parks, E.S., Parks, W.P., Josephs, S.F., Gallo, R.C. and Wong-Staal, F. Cell 45, 637-648 (1986).
Wain-Hobson, S., Sonigo, P., Danos, O., Cole, S. and Alizon, M. Cell 40, 9-17 (1985).

**Revendications**

1) Vecteur viral ou plasmidique d'expression d'une protéine du virus HIV-2 dans une cellule infectée ou transformée par ce vecteur, caractérisé en ce qu'il comporte au moins ;
- une partie du génome d'un virus hétérologue ou d'un plasmide
- une séquence de nucléotides codant pour l'une des protéines du virus HIV-2
- ainsi que les éléments assurant l'expression de cette protéine dans des cellules eucaryotes ou procaryotes.
2) Vecteur selon la revendication 1, caractérisé en ce que la partie du génome d'un virus hétérologue est une partie du génome d'un virus choisi parmi les poxvirus, les adénovirus, les virus herpétiques et les baculovirus.

3) Vecteur selon la revendication 2, caractérisé en ce que le poxvirus est le virus de la vaccine.

4) Vecteur selon les revendications 1 à 3, caractérisé en ce qu'il comporte la séquence nucléotidique qui code pour une glycoprotéine de l'enveloppe du virus HIV-2 (gp d'enveloppe).

5) Vecteur selon la revendication 4, caractérisé en ce que la séquence codant pour l'une des gp de l'enveloppe est la séquence codant pour la gp160.

6) Vecteur selon la revendication 4, caractérisé en ce que la séquence codant pour l'une des gp est la séquence codant pour la gp120.

7) Vecteur selon la revendication 4, caractérisé en ce que la séquence codant pour l'une des gp est la séquence codant pour la gp40 ou gp32.

8) Vecteur selon les revendications 4 à 6, caractérisé en ce que le gène env a subi une mutation pour supprimer le site de clivage par les protéases entre la gp120 et la gp40.

9) Vecteur selon les revendications 1 à 3, caractérisé en ce qu'il comporte la séquence nucléotidique codant pour au moins une des protéines codées par le gène gag du virus HIV-2,

10) Vecteur selon la revendication 9, caractérisé en ce que la séquence codant pour au moins une des protéines du gène gag est la séquence codant pour la P57, la P26 ou la P16.

11) Vecteur selon les revendications 1 à 3, caractérisé en ce qu'il comporte la séquence nucléotidique codant pour la protéine F du virus HIV-2.

12) Vecteur selon la revendication 11, caractérisé en ce que la séquence codant pour la protéine F est fusionnée en phase avec une séquence d'ancrage N-terminale.

13) Vecteur selon l'une des revendications 1 à 12, caractérisé en ce que la séquence d'ADN codant pour la ou les protéine(s) du virus HIV-2 est sous la dépendance d'un promoteur d'un gène de poxvirus.

14) Vecteur selon l'une des revendications 3 à 13, caractérisé en ce que le promoteur est un promoteur d'un gène du virus de la vaccine.

15) Vecteur selon l'une des revendications 3 à 14, caractérisé en ce que la séquence d'ADN codant pour la ou les protéine(s) du virus HIV-2 est sous le contrôle du promoteur du gène de la protéine 7,5 K de la vaccine.

16) Vecteur selon l'une des revendications 3 à 15, caractérisé en ce que la séquence codant pour la ou les protéines du virus HIV-2 est clonée dans le gène TK de la vaccine.

17) ADN recombinant correspondant à un vecteur selon l'une des revendications 1 à 16.

18) Cellules infectées ou transformées par un vecteur viral ou plasmidique selon l'une des revendications 1 à 16 ou par l'ADN selon la revendication 17.

19) Culture de cellules de mammifères infectées par un vecteur viral selon l'une des revendications 1 à 16.

20) Procédé de préparation de protéines du virus HIV-2, caractérisé en ce que l'on cultive des cellules selon l'une des revendications 18 ou 19 et que l'on récupère les protéines produites.

21) Glycoprotéines d'enveloppe ou protéines du virus HIV-2 obtenues par mise en oeuvre du procédé selon la revendication 20.

22) Vaccin caractérisé en ce qu'il est constitué par un vecteur viral selon l'une des revendications 1 à 16, vivant ou inactivé, et/ou par les protéines selon la revendication 21.

23) Vaccin selon la revendication 22, caractérisé en ce qu'il comporte en outre un élément vaccinant à l'encontre du virus HIV-1.

24) Anticorps dressés contre les protéines et/ou glycoprotéines du virus HIV-2, caractérisés en ce qu'on ino cule un organisme vivant avec un vecteur selon l'une des revendications 1 à 16 ou avec les protéines ou glycoprotéines obtenues selon la revendication 21 et en ce qu'on récupère les anticorps formés après un temps déterminé.

25) Trousse de diagnostic comportant une protéine ou glycoprotéine selon la revendication 21 et/ou un anticorps selon la revendication 24.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GB-A-2 181 435 (ONCOGEN)<br>* Exemples 6-9 *<br>--- | 1-20 | C 12 N 15/00<br>C 12 N 7/00<br>C 12 N 5/00<br>C 12 P 21/02<br>A 61 K 39/21<br>A 61 K 39/395<br>G 01 N 33/569 |
| Y,D | NATURE, vol. 326, 16 avril 1987, pages 662-669; M. GUYADER et al.: "Genome organization and transactivation of the human immunodeficiency virus type 2"<br>* En entier *<br>--- | 1-20 | |
| X | WO-A-8 704 459 (INSTITUT PASTEUR)<br>* Résumé *<br>----- | 21,22,<br>24,25 | |

DOMAINES TECHNIQUES RECHERCHES (Int. Cl.4)

C 12 N
C 12 P
A 61 K

Le présent rapport a été établi pour toutes les revendications

| Lieu de la recherche | Date d'achèvement de la recherche | Examinateur |
|---|---|---|
| LA HAYE | 12-12-1988 | CUPIDO M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)